# EUROPEAN PATENT APPLICATION

(11) **EP 3 628 364 A1**
(43) Date of publication of application: **01.04.2020**
(21) Application number: 19195439.5
(22) Date of filing: 04.09.2019
(51) Int. Cl.: A61M 37/00, B29C 33/38, B29C 67/00

(54) **METHOD FOR PRODUCING TRANSDERMAL ABSORPTION SHEET**

(30) Priority: 26.09.2018 JP 2018180248
(71) Applicant: FUJIFILM Corporation, Tokyo 106-8620 (JP)
(72) Inventor: OKANO, Keio, Kanagawa, 258-8577 (JP); WAKAMATSU, Satoshi, Kanagawa, 258-8577 (JP)
(74) Representative: Klunker IP Patentanwälte PartG mbB

(57) **Abstract**

A method for producing a transdermal absorption sheet includes a step of preparing a mold (120) having a plurality of needle-like recessed portions (134) and a step portion (124) which surrounds the recessed portions (134) and is higher than a needle-like recessed portion formed surface (122), and a step of supplying a liquid material (170) from a jetting opening (156) of a filling head (150) to a region surrounded the step portion (124) of the mold (120), in which the filling head (150) includes a head main body (152) having an inner wall surface defining a supply pipe (160A), and the inner wall surface includes a tapered pipe surface defining a tapered pipe (160B) having a diameter gradually increased toward the jetting opening (156) of the head main body (152). Entrainement of bubbles in the liquid material forming the absorption sheet is suppressed.

## Description

### BACKGROUND OF THE INVENTION

### 1. Field of the Invention

The present invention relates to a method for producing a transdermal absorption sheet and particularly to a technique for supplying a liquid material for a transdermal absorption sheet.

### 2. Description of the Related Art

As a method for administering a drug or the like through a living body surface, that is, a skin or mucous membrane, a method in which a chemical is injected by using a transdermal absorption sheet on which microneedles (needle-like protruding portions) having a high aspect ratio and containing a drug are formed, and inserting the needle-like protruding portions into the skin has been used.

As a method for producing a transdermal absorption sheet, a method is known in which a polymer solution or the like is poured into a mold in which needle-like recessed portions that are inverted shapes of needle-like protruding portions are formed, to transfer the shapes. In this case, it is required that the liquid material is supplied, held and dried in a state in which a predetermined pattern shape is applied to the mold (so-called patch shape). On the other hand, considering shape transfer, a material having peelability is often used as a material for constituting the mold, and the mold inevitably has water repellency in many cases. By simply applying or drawing the liquid material to the mold having water repellency, a transdermal absorption sheet cannot be formed since the liquid material shrinks and is repelled. Various proposals are made to address this problem.

For example, JP2016-168325A discloses a method for producing a transdermal absorption sheet by supplying and drying a polymer layer forming liquid using a mold including a step portion around the region in which needle-like recessed portions are formed. In addition, JP2016-106676A discloses a method for producing a needle-like body by supplying a needle-like body forming aqueous solution to an intaglio plate, and drying and peeling off the solution.

### SUMMARY OF THE INVENTION

However, in a case where a liquid material is supplied into a mold having a step portion, bubbles may be entrained in the step portion. In a case where the bubbles rise in the liquid material and diffuse from the liquid material into the atmosphere, the liquid material is repelled and there arises a problem that a transdermal absorption sheet is not formed.

The present invention is made in consideration of these circumstances, and an object thereof is to provide a method for producing a transdermal absorption sheet capable of supplying a liquid material into a mold having a step portion while suppressing the entrainment of bubbles.

A method for producing a transdermal absorption sheet according to a first aspect of the present invention comprises: a step of preparing a mold having a plurality of needle-like recessed portions, and a step portion which surrounds the plurality of needle-like recessed portions and is higher than a needle-like recessed portion formed surface; and a step of supplying a liquid material for a transdermal absorption sheet from a jetting opening of a filling head to a region surrounded the step portion of the mold, in which the filling head includes a head main body having an inner wall surface defining a supply pipe, and the inner wall surface includes a tapered pipe surface defining a tapered pipe having a diameter gradually increased toward the jetting opening of the head main body.

According to the first aspect, it is possible to suppress the entrainment of bubbles in the liquid material.

In the method for producing a transdermal absorption sheet according to a second aspect of the present invention, a diameter of a tip end of the head main body is smaller than an inner diameter of the step portion. According to the second aspect, even in a case where the filling head and the mold are separated from each other, it is possible to fix the liquid material in the step portion.

In the method for producing a transdermal absorption sheet according to a third aspect of the present invention, in the step of the supplying, a supply end position of the liquid material from the filling head is further separated from the mold than a supply start position of the liquid material from the filling head. According to the third aspect, even in a case where the filling head and the mold are misaligned, it is possible to fix the liquid material in the step portion.

In the method for producing a transdermal absorption sheet according to a fourth aspect of the present invention, while the filling head is moved from the start position to the end position, the liquid material is continuously supplied. According to the fourth aspect, it is possible to continuously supply the liquid material.

In the method for producing a transdermal absorption sheet according to a fifth aspect of the present invention, while the filling head is moved from the start position to the end position, the liquid material is intermittently supplied. According to the fifth aspect, it is possible to intermittently supply the liquid material.

In the method for producing a transdermal absorption sheet according to a sixth aspect of the present invention, the head main body has a tapered surface having a diameter gradually decreased toward a tip end of the head main body. According to the sixth aspect, even in a case where the filling head and the mold are misaligned, it is possible to allow the liquid material to reach the step portion.

The method for producing a transdermal absorption sheet according to a seventh aspect of the present invention further comprises: a step of performing suction from a surface of the mold opposite to the needle-like recessed portion formed surface. According to the seventh aspect, it is possible to fill the needle-like recessed portions with the liquid material.

The method for producing a transdermal absorption sheet according to an eighth aspect of the present invention further comprises: a step of forming a transdermal absorption sheet by drying the liquid material and separating the formed transdermal absorption sheet from the mold after the step of the supplying. According to the eighth aspect, it is possible to form a transdermal absorption sheet.

In the method for producing a transdermal absorption sheet according to a ninth aspect of the present invention, the liquid material is a polymer solution. According to the ninth aspect, it is possible to apply the polymer solution as the liquid material for a transdermal absorption sheet.

The method for producing a transdermal absorption sheet according to a tenth aspect of the present invention further comprises: a step of forming a drug layer including a drug at a tip end of the needle-like recessed portion before the step of the supplying. According to the tenth aspect, it is possible to produce a transdermal absorption sheet having a two-layer structure.

In the method for producing a transdermal absorption sheet according to an eleventh aspect of the present invention, the mold is formed of silicone resin. According to the eleventh aspect, the silicone resin is durable against transfer by repeated pressurizing and has good peelability.

In the method for producing a transdermal absorption sheet according to a twelfth aspect of the present invention, the step portion circumferentially surrounds the plurality of needle-like recessed portions, and the head main body is cylindrical. According to the twelfth aspect, it is possible to stably fix the liquid material in the circumferential step portion. The cylindrical head main body makes it easy to spread the liquid material concentrically.

According to the aspects of the present invention, it is possible to suppress the entrainment of bubbles in the liquid material.

### BRIEF DESCRIPTION OF THE DRAWINGS

Fig. 1 is a perspective view showing an example of a transdermal absorption sheet.
Fig. 2 is a perspective view showing an example of a mold.
Fig. 3 is a cross-sectional view taken along line III-III in Fig. 2.
Fig. 4 is a perspective view showing a filling head.
Fig. 5 is a view for illustrating a step of supplying a liquid material into a mold using a filling head according to a first embodiment.
Fig. 6 is a view for illustrating the step of supplying the liquid material into the mold using the filling head according to the first embodiment.
Fig. 7 is a view for illustrating the step of supplying the liquid material into the mold using the filling head according to the first embodiment.
Fig. 8 is a view for illustrating the step of supplying the liquid material into the mold using the filling head according to the first embodiment.
Fig. 9 is an illustration for illustrating a spreading direction of the liquid material in a case where the filling head according to the first embodiment is used.
Fig. 10 is a view for illustrating the step of supplying the liquid material into the mold using the filling head according to the first embodiment.
Fig. 11 is a view for illustrating the step of supplying the liquid material into the mold using the filling head according to the first embodiment.
Fig. 12 is a view for illustrating a step of producing a transdermal absorption sheet.
Fig. 13 is a view for illustrating the step of producing the transdermal absorption sheet.
Fig. 14 is a view for illustrating the step of producing the transdermal absorption sheet.
Fig. 15 is a view for illustrating a step of supplying the liquid material into the mold using a filling head according to a second embodiment.
Fig. 16 is a view for illustrating a state in which the filling head according to the first embodiment is separated from the mold.
Fig. 17 is a view for illustrating the step of supplying the liquid material into the mold using the filling head according to the second embodiment.
Fig. 18 is a view for illustrating the step of supplying the liquid material into the mold using the filling head according to the second embodiment.
Fig. 19 is a view for illustrating a modified example of the step of supplying the liquid material into the mold using the filling head according to the second embodiment.
Fig. 20 is a view for illustrating the modified example of the step of supplying the liquid material into the mold using the filling head according to the second embodiment.
Fig. 21 is a view for illustrating a step of supplying the liquid material into the mold using a filling head according to a third embodiment.
Fig. 22 is a view for illustrating a state in which the liquid material is supplied from the filling head according to the second embodiment.
Fig. 23 is a view for illustrating the step of supplying the liquid material into the mold using a filling head according to the third embodiment.

### DESCRIPTION OF THE PREFERRED EMBODIMENTS

Hereinafter, preferable embodiments of the present invention is described below in accordance with the accompanying drawings. The present invention is described using the following preferable embodiments. However, many techniques may be used to vary the embodiments without departing from the scope of the present invention, and embodiments other than the present embodiments may be used. Thus, all the variations within the scope of the present invention are included in the claims.

In the drawings, components designated by the same reference numeral are similar components with similar functions. In addition, in the present specification, in a case where a numerical range is described using "to", numerical values for an upper limit and a lower limit illustrated with "to" are also included in the numerical range.

### Transdermal Absorption Sheet

An example of a transdermal absorption sheet (microneedle array) will be described.

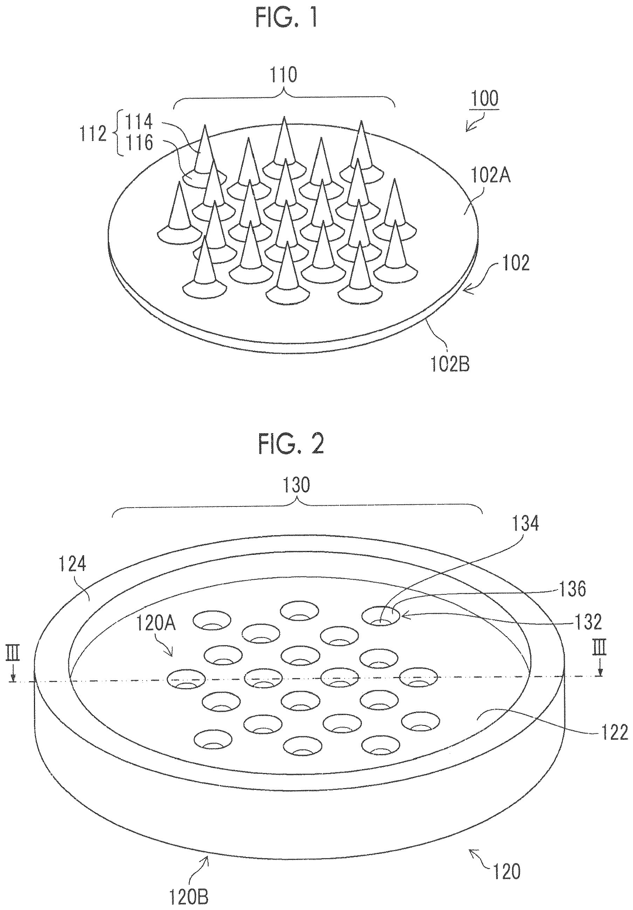

Fig. 1 is a perspective view showing an example of a transdermal absorption sheet 100. The transdermal absorption sheet 100 according to the embodiment corresponds to a patch for one dose. The transdermal absorption sheet 100 includes a sheet-like base 102 having a first surface 102A and a second surface 102B facing each other, and a protruding pattern 110.

The sheet-like shape means a flat shape as a whole, which is thinner than the two facing first surface 102A and second surface 102B having a large area, and the first surface 102A and the second surface 102B do not have to be completely flat. In addition, the base 102 shown in Fig. 1 is circular in plan view, but may be rectangular, polygonal, elliptical or the like.

The protruding pattern 110 is constituted of a plurality of needle-like protruding portions 112 constituted to include a drug. The needle-like protruding portions 112 are provided on the first surface 102 A. The needle-like protruding portion 112 includes a needle portion 114 and a frustum portion 116 connecting the needle portion 114 and the base 102.

On the first surface 102A of the transdermal absorption sheet 100, a plurality of the frustum portions 116 are arranged. The frustum portion 116 has two bottoms and has a three-dimensional structure surrounded by a pyramidal surface. A bottom having a wide area (lower bottom) of the two bottoms of the frustum portion 116 is connected to the base 102. A bottom having a narrow area (upper bottom) of the two bottoms of the frustum portion 116 is connected to the needle portion 114. Out of the two bottoms of the frustum portion 116, the area of the bottom in the direction away from the base 102 is small.

The needle portion 114 has a bottom having a wide area and has a shape in which the tip end distant from the bottom has the narrowest area. Since the bottom having a wide area of the needle portion 114 is connected to the upper bottom of the frustum portion 116, the needle portion 114 has a shape tapered in a direction away from frustum portion 116. The needle-like protruding portion 112 constituted of the needle portion 114 and the frustum portion 116 has a tapered shape from the base 102 toward the tip end as a whole. A plurality of the needle-like protruding portions 112 of 4 to 2500 are provided on the base 102. However, the embodiment is not limited to this number.

In Fig. 1, the frustum portion 116 has a truncated cone shape and the needle portion 114 has a conical shape. Depending on the degree of insertion of the needle portion 114 into the skin, the shape of the tip end of the needle portion 114 can be appropriately changed to a curved surface, a flat surface, or the like with a radius of curvature of 0.01 µm or more and 50 µm or less.

### Mold

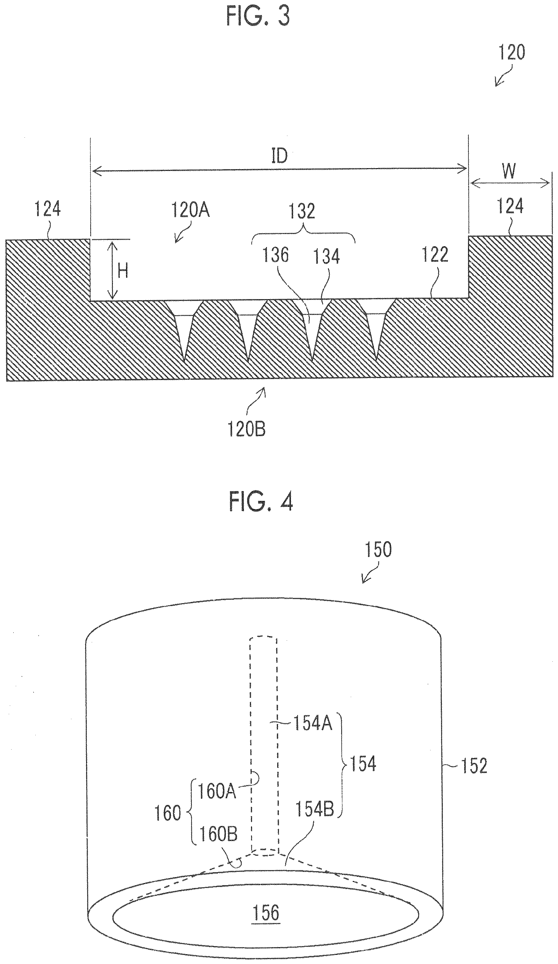

Fig. 2 is a perspective view showing an example of a mold 120 for producing (molding) the transdermal absorption sheet 100. In addition, Fig. 3 is a cross-sectional view taken along line III-III in Fig. 2. The mold 120 has a first surface 120A, a second surface 120B, and has a flat portion 122, a step portion 124, and a recessed pattern 130 on the side of the first surface 120A. The flat portion 122 becomes a needle-like recessed portion formed surface.

The recessed pattern 130 is constituted of a plurality of needle-like recessed portions 132 provided on the flat portion 122. The needle-like recessed portion 132 has a shape corresponding to the needle-like protruding portion 112 of the transdermal absorption sheet 100 and is constituted of a tip end recessed portion 134 corresponding to the needle portion 114 and a cup portion 136 corresponding to the frustum portion 116.

The tip end recessed portion 134 has a shape tapered in the depth direction (thickness direction) of the mold 120. The tip end recessed portion 134 can be formed to have a diameter of 150 µm to 500 µm and a height of 150 µm to 2000 µm. In addition, the cup portion 136 has an opening on the side of the first surface 120A of the mold 120 and has a shape that narrows in the depth direction toward the second surface 120B of the mold 120. The cup portion 136 is connected to the tip end recessed portion 134 at the narrowest portion. The cup portion 136 can be formed to have a diameter of 500 µm to 1000 µm and a height of 100 µm to 500 µm.

The shape of the needle-like recessed portion 132 is not limited to this example. Between the tip end recessed portion 134 and the cup portion 136, an intermediate recessed portion having a constant width in the depth direction, such as a column, a square pole, or a polygonal pole, may be provided. In addition, a through-hole passing through the mold 120 and reaching the second surface 120B may be formed at the tip end of the tapered shape. The arrangement, pitch, number, and the like of the needle-like recessed portions 132 are determined according to the arrangement, pitch, number, and the like of the needle-like protruding portions 112 required for the transdermal absorption sheet 100.

The flat portion 122, which is a needle-like recessed portion formed surface, has a flat or substantially flat shape corresponding to the base 102 of the transdermal absorption sheet 100. The step portion 124 circumferentially surrounds the recessed pattern 130 and is higher than the flat portion 122. The term "higher than the flat portion 122" means that the distance from the second surface to the step portion 124 is larger than the distance from the second surface 120B to the flat portion 122 using the second surface 120B as the reference. Since the second surface 120B is flat, the distance to the top portion of the step portion 124 is larger than the distance to the flat portion 122 using the second surface 120B as the reference. A difference between the two distances is a height H. The width W of the step portion 124 in the radial direction of the mold 120 is preferably 1.5 mm or more. In the embodiment, the case where the step portion 124 circumferentially surrounds the recessed pattern 130 is shown, but the shape of the step portion 124 is not particularly limited. For example, the step portion 124 may surround the recessed pattern 130 in a polygonal shape, or the step portion 124 may surround the recessed pattern 130 in a curved form with multiple curvatures. The shape of the step portion 124 is determined in consideration of the amount of liquid material, the physical properties (surface tension, viscosity, and wettability with respect to the mold), and the like. In a case where the step portion 124 is circular, the liquid material is fixed at the same strength all around the step portion 124 and thus the liquid material can be stably fixed. For example, in a case where the step portion 124 is rectangular or the step portion 124 has a curvature higher than the other portions, the fixation between the liquid material and the step portion 124 is weakened. There is a concern that the movement of the contact line described later may occur at the weakly fixed portion.

In the embodiment, since the angle formed by the flat portion 122 and the step portion 124 is 90 degrees, an inner diameter ID of the step portion 124 matches the diameter of the flat portion 122.

The material used for the mold 120 is preferably a material having flexibility and more preferably a material having high gas permeability. The oxygen permeability, which is representative of gas permeability, is preferably more than 1 × 10⁻¹² (mL/s·m·Pa) and even more preferably more than 1 × 10⁻¹⁰ (mL/s·m·Pa). By producing the mold 120 using a material with high gas permeability, the liquid material filled in the needle-like recessed portion 132 can be sucked by suction from the second surface 120 B of the mold 120, and the filling into the needle-like recessed portion 132 can be promoted. In addition, the air present in the needle-like recessed portion 132 can be removed from the second surface 120B side. Thus, it is possible to produce the transdermal absorption sheet 100 with few defects.

Specifically, examples of such materials include materials obtained by melting a silicone resin (for example, SYLGARD 184 (registered trademark) manufactured by Dow Corning Toray Co., Ltd. or 1310ST manufactured by Shin-Etsu Chemical Co., Ltd.), an ultraviolet curable resin, a polystyrene resin, a polymethylmethacrylate resin, an epoxy resin, a polyethylene terephthalate resin, a polyoxymethylene resin, a polytetrafluoroethylene resin, a polyethylene resin, a phenolic resin, and a urethane resin, and materials obtained by dissolving any of above resins into a solvent.

Among these, the silicone resin is durable against transfer by repeated pressurizing and has good peelability from the material. The silicone resin can be suitably used as the material for the mold 120.

### First Embodiment

A method of producing a transdermal absorption sheet according to a first embodiment will be described with reference to the drawings. Fig. 4 is a perspective view showing a filling head to be applied to the production method according to the first embodiment. A filling head 150 has a generally cylindrical head main body 152. A jetting opening 156 for jetting the liquid material is formed at the tip end of the head main body 152. The head main body 152 has an inner wall surface 160 that defines a supply pipe 154 for allowing the passing of the liquid material. The supply pipe 154 includes a cylindrical pipe 154A having a constant inner diameter, and a tapered pipe 154B of which the diameter is gradually increased toward the jetting opening 156. The inner wall surface 160 has a cylindrical pipe surface 160A that defines the cylindrical pipe 154A and a tapered pipe surface 160B that defines the tapered pipe 154B. The cylindrical pipe 154A and the tapered pipe 154B are continuously formed, and the tapered pipe 154B is continuous to the jetting opening 156. Since the diameter of the tapered pipe 154B is gradually increased toward the jetting opening 156, the inner diameter of the tapered pipe 154B on the side of the jetting opening 156 (the opening diameter of the tapered pipe 154B) is larger than the inner diameter on the side of the cylindrical pipe 154A. The central axis of the supply pipe 154 matches with the central axis of the head main body 152.

The term "constant inner diameter" includes a case where the inner diameter is completely constant and a case where the inner diameter is substantially constant. The inner diameter, the length, and the like of the cylindrical pipe 154A are appropriately determined. Therefore, the term "cylindrical" includes being perfectly cylindrical and being substantially cylindrical. Although the cylindrical filling head 150 is shown in the embodiment, the shape of the filling head 150 is not particularly limited.

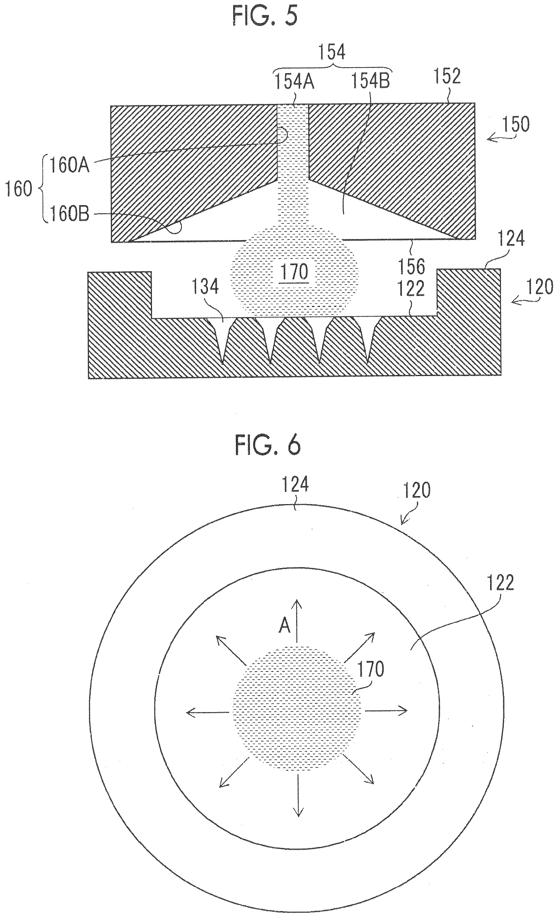

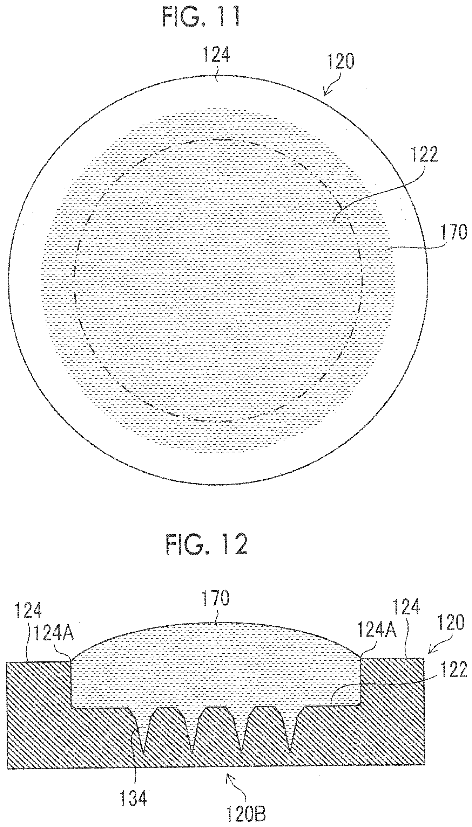

Figs. 5 to 11 are views for illustrating a step of supplying a liquid material for a transdermal absorption sheet to the mold 120 using the filling head of the first embodiment. Figs. 12 to 14 are views for illustrating a step of producing a transdermal absorption sheet.

As shown in Fig. 5, the mold 120 including the step portion 124 is prepared. The filling head 150 and the mold 120 are aligned. In the alignment, the head main body 152 and the mold 120 are moved relative to each other, and the central position of the head main body 152 and the central position of the step portion 124 of the mold 120 are aligned. The alignment is performed using, for example, a drive mechanism that moves in the XYZ directions (not shown). At least one of the filling head 150 or the mold 120 may be moved. The filling head 150 is moved to a start position where the supply of the liquid material 170 is started. The start position is determined in advance in consideration of the relative position to the mold 120.

The supply of the liquid material 170 for a transdermal absorption sheet is started. The liquid material 170 which has passed through the cylindrical pipe 154A is supplied from the jetting opening 156 of the filling head 150 to the region surrounded by the step portion 124 of the mold 120. In a case where the center of the filling head 150 matches with the center of the mold 120, the liquid material 170 is supplied to the center of the mold 120. As shown in Fig. 6, the liquid material 170 spreads in a direction from the center of the mold 120 toward the step portion 124 as indicated by an arrow A (hereinafter, referred to as "radial direction"). However, since the spreading speed of the liquid material 170 is not constant in all directions, it is difficult for the liquid material 170 to spread completely concentrically.

As shown in Fig. 5, the opening diameter of the tapered pipe 154B is larger than the inner diameter of the step portion 124 of the mold 120. The outer circumferential diameter (outer diameter) of the filling head 150 is larger than the inner diameter of the step portion 124 and smaller than the outer diameter of the mold 120. The outer diameter of the filling head 150 is substantially constant along the supply direction of the liquid material 170.

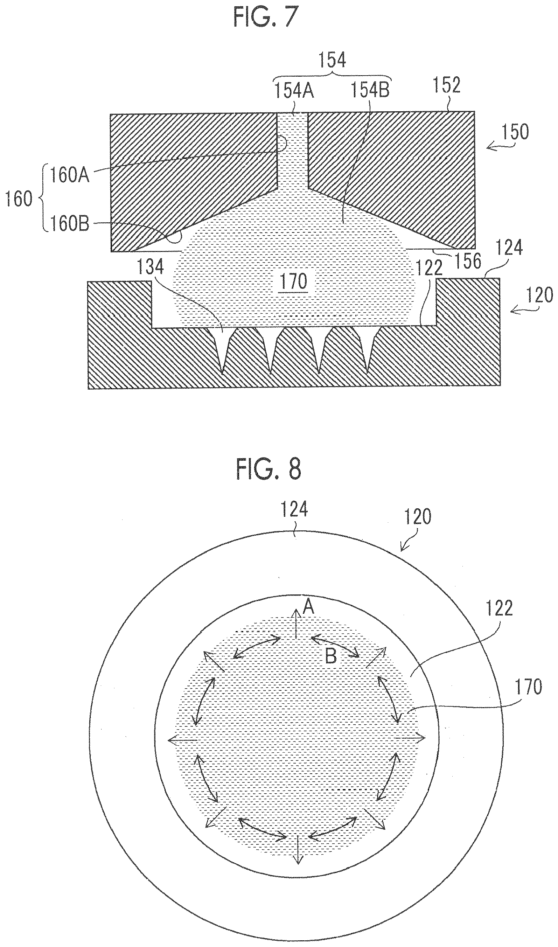

As shown in Fig. 7, the liquid material 170 is continuously supplied until the required amount is obtained. Before reaching the step portion 124, the liquid material 170 comes into contact with the tapered pipe surface 160B. In a case where the filling head 150 is brought close to the mold 120, the gap between the filling head 150 and the mold 120 is narrowed in a direction from the center of the mold 120 to the step portion 124 on the tapered pipe surface 160B.

As shown in Fig. 8, in a case where the liquid material 170 is supplied, the liquid material 170 tends to flow out in the radial direction indicated by the arrow A. On the other hand, the liquid material 170 also tends to flow out in the circumferential direction indicated by an arrow B.

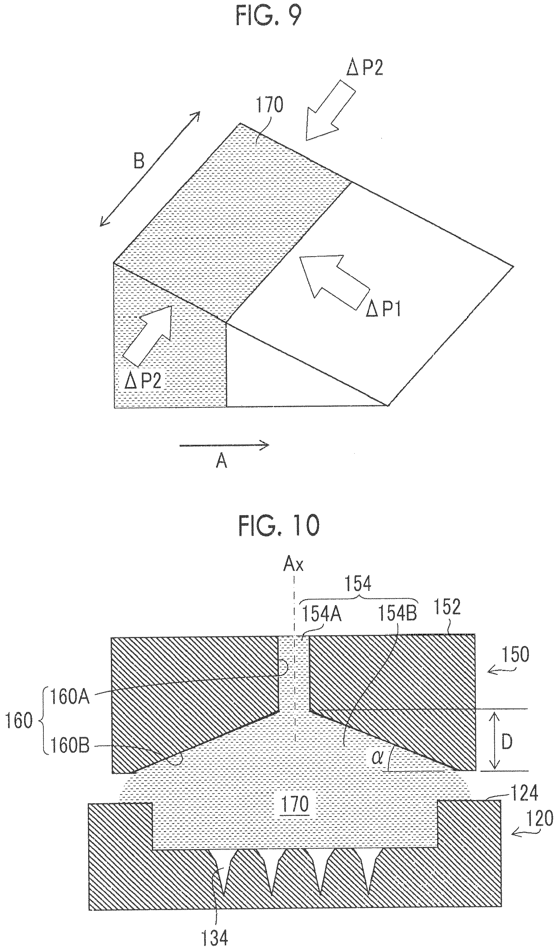

Fig. 9 is an illustration for illustrating a spreading direction of the liquid material 170 in a case where the filling head 150 according to the first embodiment is used. As shown in Fig. 9, the gap between the filling head 150 and the mold 120 is gradually decreased toward the step portion 124 (not shown). Therefore, as the liquid material 170 is moved in the radial direction indicated by the arrow A, a pressure loss ΔP1 that the liquid material 170 receives is increased. The liquid material 170 is less likely to receive a pressure loss ΔP2, and therefore, easily flows in the circumferential direction indicated by the arrow B. The pressure loss ΔP1 is larger than the pressure loss ΔP2. Since the jetting distribution of the liquid material 170 in the circumferential direction is adjusted, the liquid material 170 spreads in the radial direction while uniformly spreading concentrically.

As shown in Figs. 10 and 11, since the liquid material 170 supplied from the filling head 150 spreads concentrically, the liquid material 170 reaches the step portion 124 simultaneously. In the case where the liquid material 170 reaches the step portion simultaneously, the entrainment of bubbles in the liquid material 170 is prevented. Here, reaching simultaneously include complete simultaneousness and some time difference as long as no bubbles are formed. The supplied liquid material 170 flows in the gap between the filling head 150 and the mold 120 until the liquid material rides on the step portion 124. The liquid material 170 may ride on the step portion 124 beyond the head main body 152. It is preferable that the liquid material 170 does not overcome the step portion 124. In a case where the required amount of the liquid material 170 is supplied, the supply of the liquid material 170 from the filling head 150 to the mold 120 is ended.

The filling head 150 and the mold 120 at the end position where the supply of the liquid material 170 is ended are relatively separated from each other. The liquid material 170 is transferred to the mold 120. In the first embodiment, the start position and the end position are the same.

Regarding a taper angle α of the filling head 150, as shown in Fig. 10, as the taper angle α increases, a gradient of the pressure loss ΔP1 in the radial direction increases. Accordingly, the jetting distribution of the liquid material 170 in the circumferential direction is more reliably adjusted. On the other hand, as the taper angle α increases, the depth D of the tapered pipe surface 160B increases. Therefore, the gap between the filling head 150 and the step portion 124 of the mold 120 becomes large. As the gap increases, the amount of the liquid material 170 filling the gap between the filling head 150 and the mold 120 increases, and the amount of the liquid material 170 to be transferred to the mold 120 increases. It is preferable to adjust the taper angle α and determine the taper angle α within a range in which the amount of the liquid material 170 does not become too large. The taper angle α is an angle formed between the surface vertically crossing a central axis Ax of the filling head 150 and the tapered pipe surface 160B.

As shown in Fig. 12, the liquid material 170 is repelled by the step portion 124 of the mold 120 and contracts due to surface tension. The liquid material 170 is fixed (also referred to as pinning) at the position of a corner portion 124A on the inner diameter side of the step portion 124.

In order to fill the needle-like recessed portions 132 constituting the recessed pattern 130 with the liquid material 170, it is preferable to perform suction from the second surface 120B, which is the surface of the mold 120 opposite to the flat portion 122.

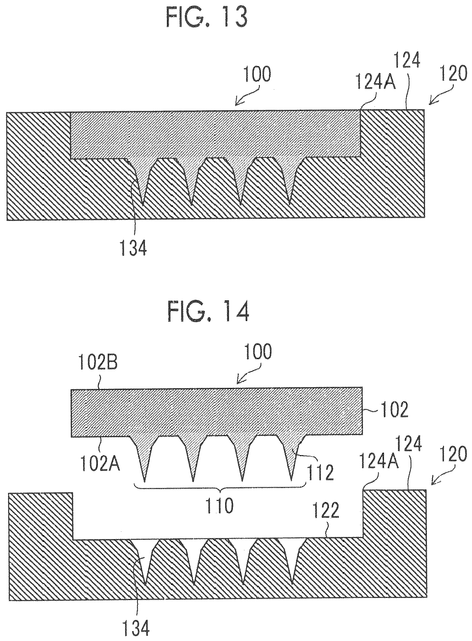

As shown in Fig. 13, the liquid material 170 is dried to form the transdermal absorption sheet 100 in the mold 120. In Fig. 12, since the liquid material 170 is fixed at the corner portion 124A, even after drying, the contact line between the liquid material 170 and the air-liquid interface is not moved. It is possible to form the transdermal absorption sheet 100 having a desired shape.

As shown in Fig. 14, the transdermal absorption sheet 100 formed in the mold 120 is separated from the mold 120. The separation method is not particularly limited. For example, the transdermal absorption sheet 100 is separated from mold 120 by adsorbing the second surface 102B of the transdermal absorption sheet 100 with a suction pad and moving the suction pad away from the mold 120.

In the embodiment, the case where the liquid material 170 is supplied to the region surrounded by the step portion 124 of the mold 120, the needle-like recessed portions 132 of the receded pattern 130 are filled with the liquid material, and the liquid material is dried to form the transdermal absorption sheet 100 is described, but the embodiment is not limited thereto.

For example, a drug layer can be formed on the tip end side of each of the needle-like recessed portions 132 of the recessed pattern 130 before the liquid material 170 is supplied. After the drug layer is formed, a transdermal absorption sheet 100 having a two-layer structure can be produced by supplying the liquid material 170 not including a drug. The solidified drug layer can suppress diffusion of the drug layer into the liquid material 170.

As the liquid material 170, a polymer solution is preferable. It is preferable to use a water-soluble material as the material of the polymer solution. As the material of a resin polymer of the polymer solution, it is preferable to use a biocompatible resin. As such a resin, it is preferable to use saccharides such as glucose, maltose, pullulan, sodium chondroitin sulfate, sodium hyaluronate, and hydroxyethyl starch, proteins such as gelatin, or a biodegradable polymer such as polylactic acid or a lactic acid-glycolic acid copolymer. In a case where the transdermal absorption sheet 100 is separated from the mold 120, since the transdermal absorption sheet 100 can be released using a base material (not shown), these materials can be suitably used. Although the concentration varies depending on the material, the concentration of the resin polymer in the polymer solution not including a drug is preferably 10% by mass or more and 50% by mass or less. In addition, a solvent used for the polymer solution may be other than hot water as long as the solvent has volatility, and alcohols such as ethanol can be used.

The above-mentioned polymer solution including a predetermined amount of drug can be applied as a liquid material for forming a drug layer. Whether or not a predetermined amount of drug is included is determined by whether or not the drug effect can be exerted in a case where the body surface is punctured. Therefore, including a predetermined amount of drug means including the drug in an amount that exerts a medicinal effect in a case where the body surface is punctured.

The drug is not limited as long as the drug accomplishes the functions of the drug. Particularly, the drug is preferably selected from peptide, protein, nucleic acid, polysaccharide, a vaccine, a medical compound belonging to a water-soluble low-molecular-weight compound, or a cosmetic component.

As the method for preparing the polymer solution, in a case of using a water-soluble polymer (such as gelatin), a water-soluble powder may be dissolved into water, and after the dissolution, a drug may be added to the solution or a water-soluble polymer powder may be poured and dissolved in a liquid containing a drug dissolved therein. In a case where the polymer is difficult to dissolve into water, the polymer may be dissolved by heating. The temperature may be selected, as appropriate, depending on the kind of the polymer material, but the solution is preferably heated to a temperature of about 20°C or higher and 40°C or lower as required. The viscosity of the polymer solution is preferably 200 mPa·s or less, and more preferably 50 mPa·s or less for the polymer solution including a drug. The viscosity of the polymer solution is preferably 2000 mPa·s or less and more preferably 500 mPa·s or less for the solution not including a drug. Appropriate adjustment of the viscosity of the polymer solution facilitates injection of the polymer solution into the recessed pattern 130 of the mold 120 (refer to Fig. 2). The viscosity of the polymer solution can be measured with a capillary viscometer, a falling ball type viscometer, a rotary viscometer, or a vibration type viscometer. It is preferable to remove the gas of the polymer solution by a degassing device or the like before the polymer solution is supplied to the mold 120.

### Second Embodiment

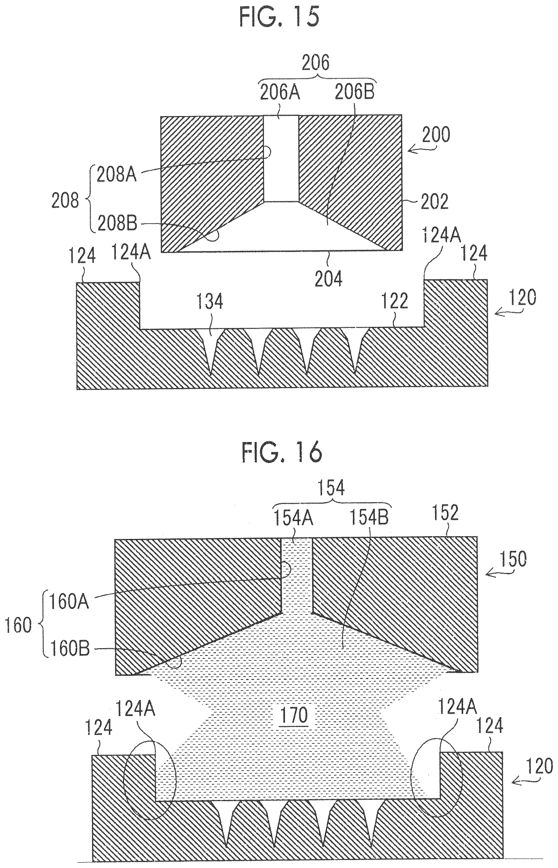

A method of producing a transdermal absorption sheet according to a second embodiment will be described with reference to Figs. 15 to 20. In the second and subsequent embodiments, the description of items common to the first embodiment may be omitted.

As shown in Fig. 15, the second embodiment is different from the first embodiment in that the relationship between the sizes of a filling head 200 and the mold 120 is different. As shown in Fig. 15, the outer diameter of the filling head 200 is smaller than the inner diameter of the step portion 124 of the mold 120. The inner diameter of a jetting opening 204 (the opening diameter of a tapered pipe 206B) is also smaller than the inner diameter of the step portion 124. The outer diameter of the filling head 200 is substantially constant along the supply direction of the liquid material 170.

The filling head 200 includes a head main body 202 as the filling head 150. The head main body 202 includes a jetting opening 204, and an inner wall surface 208 that defines a supply pipe 206. The supply pipe 206 includes a cylindrical pipe 206A, and a tapered pipe 206B. The inner wall surface 208 has a cylindrical pipe surface 208A that defines the cylindrical pipe 206A and a tapered pipe surface 208B that defines the tapered pipe 206B.

Fig. 16 is a view for illustrating a state in which the filling head 150 according to the first embodiment is relatively separated from the mold 120. As shown in Fig. 16, in a case where the opening diameter of the tapered pipe 154B is larger than the inner diameter of the step portion 124 of the mold 120, at the separation of the filling head 150, the liquid material 170 is drawn near the center. The phenomenon that the liquid material 170 is not fixed at the corner portion 124A of the step portion 124 occurs. For example, as indicated by the right circle, the liquid material 170 comes out from the corner portion 124A and the contact line of the air-liquid interface of the liquid material 170 is moved to the flat portion 122. In addition, as indicated by the left circle, the liquid material 170 comes out from the corner portion 124A and the contact line of the air-liquid interface of the liquid material 170 is moved to the middle of the step portion 124. Thus, in a case where the liquid material 170 is not fixed at the corner portion 124A, the transdermal absorption sheet 100 cannot maintain the correct shape.

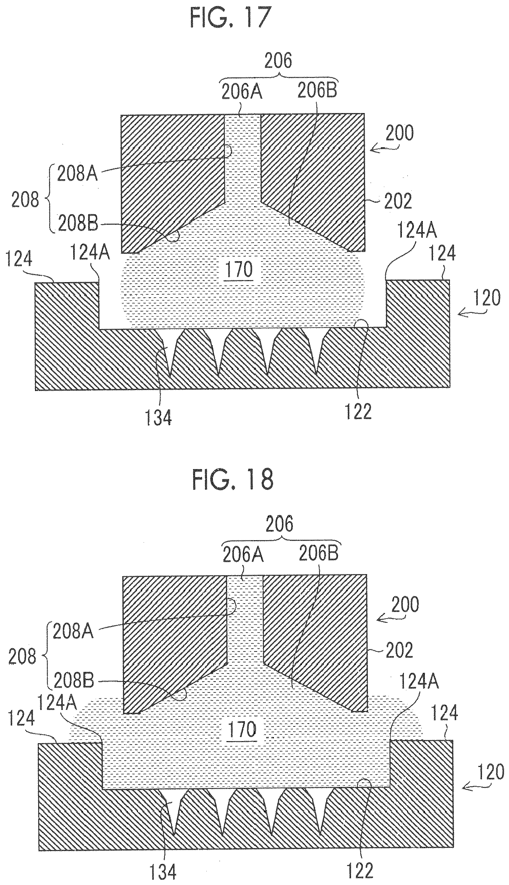

In the second embodiment, as shown in Fig. 17, the outer diameter of the filling head 200 is smaller than the inner diameter of the step portion 124 of the mold 120. The liquid material 170 flows in the gap between the head main body 202 of the filling head 200 and the mold 120. The liquid material 170 passes through the gap between the head main body 202 and the mold 120, and tends to spread toward the step portion 124 beyond the head main body 202.

As shown in Fig. 18, the supplied liquid material 170 flows until the liquid material rides on the step portion 124, and some of the liquid material 170 flows along the outer shape of the head main body 202 in a direction away from mold 120. A large amount of the liquid material 170 is present between the step portion 124 and the head main body 202 as compared to the first embodiment.

In a case where the supply of the liquid material 170 is ended, the filling head 200 and mold 120 are relatively separated from each other. The liquid material 170 is transferred to the mold 120. As shown in Fig. 18, the liquid material 170 has a distribution that becomes thicker on the side of the step portion 124 than near the center. Therefore, even in a case where the liquid material 170 is drawn near the center during the separation, the liquid material 170 can be fixed at the corner portion 124A of the step portion 124. The transdermal absorption sheet 100 can maintain the correct shape.

In a case where the outer diameter of the head main body 202 of the filling head 200 is extremely smaller than the inner diameter of the step portion 124, there is a concern that the liquid material 170 may not reach the step portion 124 simultaneously. Therefore, it is preferable to make the filling head 200 small enough to allow the liquid material 170 supplied from the filling head 200 to sufficiently reach the step portion 124 in consideration of the error of each dimension and the operation error.

### Modified Example

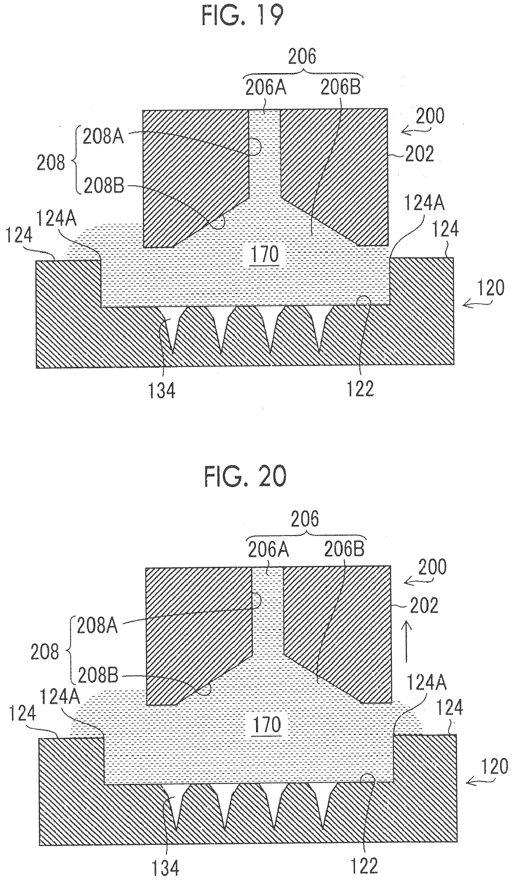

Next, a modified example of the second embodiment will be described. Fig. 19 shows a state in which the liquid material 170 is supplied to the mold 120 from the filling head 200 at the supply start position. In Fig. 19, the center positions of the filling head 200 and the mold 120 do not match due to a mechanical error or the like.

The liquid material 170 is gathered on the side with a large gap between the filling head 200 and the step portion 124. On the side with a small gap between the filling head 200 and the step portion 124, the amount of the liquid material 170 is small and thin. In a case where the supply of the liquid material 170 is ended and the filling head 150 and the mold 120 are relatively separated from each other in this state, there is a concern that the liquid material 170 may not be fixed at the corner portion 124A of the step portion 124.

Therefore, as shown in Fig. 20, the supply end position of the liquid material 170 is set at a position distant from the mold 120 from the supply start position of the liquid material 170. The distance between the filling head 200 and the mold 120 is large at the end position compared to the start position. Since the liquid material 170 is supplied while the filling head 200 is moved from the start position to the end position, a larger amount of the liquid material 170 is supplied between the filling head 200 and the step portion 124.

In the state shown in Fig. 20, even in the case where the supply of the liquid material 170 is ended and the filling head 150 and the mold 120 are relatively separated from each other, the liquid material 170 can be fixed at the corner portion 124A of the step portion 124.

The liquid material 170 can be supplied to the mold 120 continuously while the filling head 200 is moved from the start position to the end position. The supply of the liquid material 170 is not interrupted until the filling head 200 reaches the end position from the start position. In a case of supplying the liquid material 170 continuously, the supply rate from the start position to the end position may or may not be constant. The supply of the liquid material 170 is ended at the end position.

In addition, while the filling head 200 is moved from the start position to the end position, the liquid material 170 can be supplied to the mold 120 intermittently. The interruption of the supply of the liquid material 170 is allowed until the filling head 200 reaches the end position from the start position. For example, the liquid material 170 is supplied from the filling head 200 at the start position by a predetermined amount. The supply of the liquid material 170 is interrupted and the filling head 200 is moved to the end position. The liquid material 170 is supplied from the filling head 200 at the end position by a predetermined amount. The supply of the liquid material 170 is ended at the end position.

In a case where the supply of the liquid material 170 is ended, the filling head 200 and the mold 120 are relatively moved from each other. The liquid material 170 is transferred to the mold. The liquid material 170 is fixed at the corner portion 124A of the step portion 124. In the second embodiment, it is preferable that the shape of the step portion 124 surrounding the recessed pattern 130 and the outer shape of the filling head 200 are similar. The similar shape facilitates the liquid material 170 to reach the step portion 124 simultaneously or nearly simultaneously.

As in the first embodiment, by drying the liquid material 170, the transdermal absorption sheet 100 is formed, and the transdermal absorption sheet 100 is separated from the mold 120.

The modified example can be applied to the case where the filling head 150 of the first embodiment is used.

### Third Embodiment

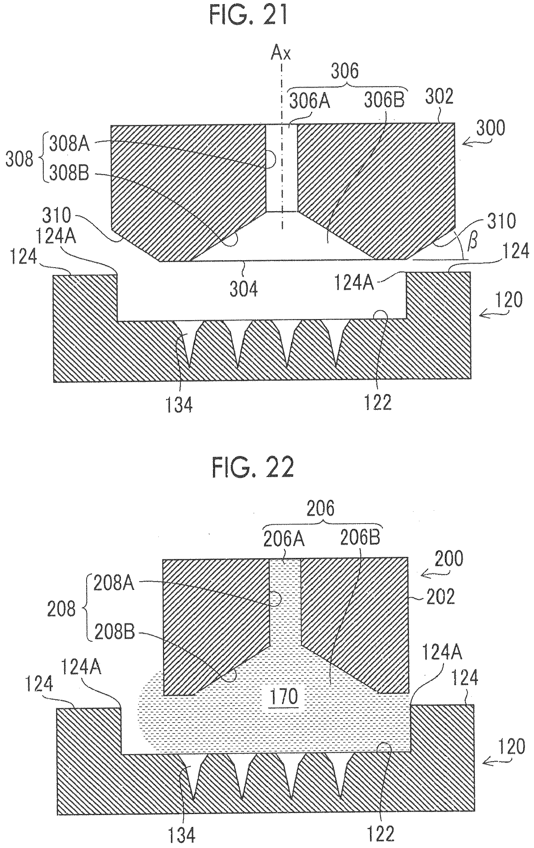

A method of producing a transdermal absorption sheet according to a third embodiment will be described with reference to Figs. 21 to 23. In the third embodiment, as shown in Fig. 21, the shape of a filling head 300 is different from the shape of the filling head 150 of the first embodiment and the shape of the filling head 200 of the second embodiment. As shown in Fig. 21, the filling head 300 includes a head main body 302. The head main body 302 includes a jetting opening 304, and an inner wall surface 308 that defines a supply pipe 306. The supply pipe 306 includes a cylindrical pipe 306A, and a tapered pipe 306B. An inner wall surface 308 has a cylindrical pipe surface 308A that defines the cylindrical pipe 306A and a tapered pipe surface 308B that defines the tapered pipe 306B. The head main body 302 has a tapered surface 310 having a diameter gradually decreased toward the tip end of the head main body 302. The tip end outer diameter of the tapered surface 310 of the filling head 300 is smaller than the inner diameter of the step portion 124. The outer diameter of the filling head 300 excluding the tapered surface 310 is larger than the inner diameter of the step portion 124. The inner diameter of the jetting opening 304 (the opening diameter of the tapered pipe 306B) is also smaller than the inner diameter of the step portion 124. A taper angle β of the tapered surface 310 can be appropriately determined as long as the liquid material 170 reaches the step portion 124. The taper angle β is an angle formed between the surface vertically crossing a central axis Ax of the filling head 300 and the tapered surface 310.

Fig. 22 shows a state in which the liquid material 170 is supplied to the mold 120 from the filling head 200 of the second embodiment. As shown in Fig. 22, in a case where the outer diameter of the filling head 200 is smaller than the inner diameter of the step portion 124, and the center positions of the filling head 200 and the mold 120 do not match, the liquid material 170 flows along the outer shape of the filling head 200 and there is a concern that the liquid material 170 may not reach the step portion 124. As a result, the transdermal absorption sheet 100 cannot maintain the correct shape.

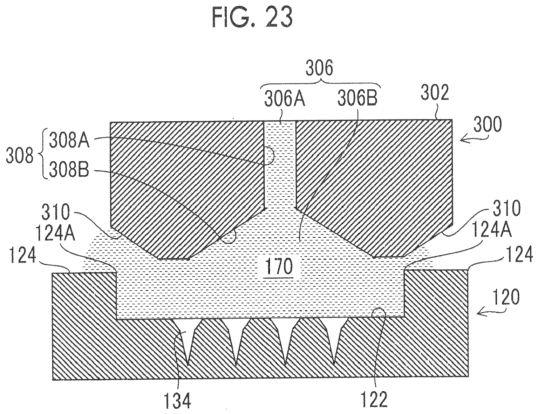

As shown in Fig. 23, the head main body 302 has the tapered surface 310. The liquid material 170 supplied from the filling head 300 reaches the tapered surface 310 of the head main body 302. The liquid material 170 spreads toward the step portion 124 by the tapered surface 310. The tapered surface 310 facilitates the liquid material 170 to reach the step portion 124. As a result, the transdermal absorption sheet 100 can maintain the correct shape. In the third embodiment, it is preferable that the shape of the step portion 124 surrounding the recessed pattern 130 and the outer shape of the filling head 300 are similar. The similar shape facilitates the liquid material 170 to reach the step portion 124 simultaneously or nearly simultaneously.

As in the first embodiment, by drying the liquid material 170, the transdermal absorption sheet 100 is formed, and the transdermal absorption sheet 100 is separated from the mold 120.

In addition, the modified example (in which the start position and the end position different arc different) of the second embodiment can be applied to a case where the filling head 300 of the third embodiment is used.

### Examples

Hereinafter, the present invention will be more specifically described using examples of the present invention. The materials, used amounts, ratios, treatment contents, treatment procedures, and the like shown in the following examples can be appropriately changed without departing from the gist of the present invention. Therefore, the scope of the present invention should not be interpreted in a limited manner based on the specific examples illustrated below.

### Preparation of Mold

A precursor for preparing a mold was prepared. The precursor was prepared by, on the surface of a smooth Ni plate having a side of 40 mm, forming protruding portions having a needle-like structure, two-dimensionally arranged in 10 columns × 10 rows, by grinding, and further, forming a cylinder shape having a height of 500 µm, centered on the middle of a two-dimensional array of 10 columns × 10 rows, by cutting. The protruding portion having a needle-like structure includes a truncated cone shape and a cone formed in the truncated cone shape. The diameter of the bottom of the truncated cone shape was 500 µm and the height of the truncated cone shape was 150 µm. The height of the cone was 500 µm. The pitch of the two-dimensionally arranged protruding portions having a needle-like structure was 1000 µm.

Three kinds of precursors with different diameters of cylindrical shapes were prepared. The diameter of the cylindrical shape of each precursor is 12 mm, 16 mm, and 20 mm.

On each precursor, a film of silicone rubber (SILAST1C-MDX4-4210, manufactured by Dow Corning Corporation) having a thickness of 0.6 mm was formed, thermally cured, and peeled off. At the center portion of the silicone rubber (the inverted article of the precursor), the protruding portions having a needle-like structure, two-dimensionally arranged in 10 columns × 10 rows, were formed. The silicone rubber was cut off to leave a planar surface with a side of 30 mm and the obtained portion was used as a mold. A surface of the mold corresponding to the wider opening of each of the needle-like protruding portions was the first surface of the mold, and the surface of the mold opposite to the first surface was the second surface.

In each mold, step portions each having inner diameters of 12 mm, 16 mm, and 20 mm corresponding to the cylindrical shape of the precursors was formed. The height of the step portion was 500 µm (0.5 mm).

### Preparation of Polymer Solution

A polymer solution was prepared as a liquid material. A solution obtained by dissolving chondroitin sulfate (manufactured by Maruha Nichiro Foods Co., Ltd.) in water and adjusting the concentration to 40% was used as a liquid material for producing a transdermal absorption sheet. After preparation, the polymer solution was exposed to a reduced pressure environment of 3 kPa for 4 minutes to perform sufficient degassing.

Hereinafter, all the preparation steps of the transdermal absorption sheet were performed under the environment of a temperature of 5°C and a relative humidity of 35 %RH.

### Supply and Drying of Polymer Solution

The polymer solution was sent by a dispenser (manufactured by Musashi Engineering, Inc.) while adjusting the jetting amount of the polymer solution and the clearance between the mold and the filling head. The polymer solution was supplied to the mold. Thereafter, it was confirmed whether or not the shape of the transdermal absorption sheet was maintained after 12 hours had passed. The evaluation was performed based on the following standards. In addition, the operation of the filling head for liquid supply was performed using a bench-top robot manufactured by Musashi Engineering, Inc.

### Experiment 1

As filling heads used for supply, three kinds of filling heads having tip end outer circumferential diameters of 13 mm, 17 mm, and 21 mm each of which are 1 mm larger than the inner diameter of the step portion of each mold were prepared. Each filling head has a tapered pipe surface (tapered structure). The opening diameter (taper opening diameter) of the tapered pipe of each filling head was 11 mm, 15 mm, and 19 mm each of which are 1 mm smaller than the inner diameter of the step portion of each mold. The depth of the tapered pipe was 0.5 mm. The diameter of the cylindrical pipe of each filling head was 5 mm. The filling head was formed of fluorocarbon resin.

The distance between the filling head and the flat portion (needle-like recessed portion formed surface) of the mold was set to 0.5 mm, and was used as the start position. The filling head is moved to the start position. The supply of the polymer solution was started from the filling head. The amount of the polymer solution supplied was such an amount that the step portion was filled with the polymer solution and the amount slightly exceeded the height of the step portion. The filling head was not moved until the supply of the polymer solution was ended. The start position and the end position were not changed.

As a comparative example, a filling head having three kinds of outer circumferential diameters not having a tapered pipe surface was prepared. Only the cylindrical pipe was provided in the filling head of the comparative example.

The evaluation was performed 20 times for each combination of the filling head and the mold, and the number of times of entrainment of bubbles immediately after the supply of the polymer solution was counted. Table 1 shows the conditions and the evaluation results.

**Table 1**

| Test No. | Inner diameter of step portion [mm] | Outer circumferential diameter of filling head [mm] | Tapered structure | Tapered opening diameter [mm] | Number of times of bubbles |
|---|---|---|---|---|---|
| 1 | 12 | 13 | No provided | - | 11 |
| 2 | 12 | 13 | Provided | 11 | 1 |
| 3 | 16 | 17 | No provided | - | 16 |
| 4 | 16 | 17 | Provided | 15 | 2 |
| 5 | 20 | 21 | No provided | - | 17 |
| 6 | 20 | 21 | Provided | 19 | 3 |

According to Test Nos. 2, 4, and 6 in Table 1, it can be understood that in a case where the polymer solution is supplied from the filling head having a tapered structure to the mold with a step portion, the number of times of entrainment of bubbles is reduced and the polymer solution can be pinned. On the other hand, according to Test Nos. 1, 3, and 5 in Table 1, in the filing head not having a tapered structure, the number of times of entrainment of bubbles was large.

### Experiment 2

In Experiment 1, it was possible to reduce the entrainment of bubbles by the filling head having a tapered structure. On the other hand, in a case where the filling head and the mold are relatively separated from each other after the supply of the polymer solution is ended, the polymer solution fixed at the corner portion of the step portion is drawn to the filling head. The polymer solution may not be fixed to the step portion and the contact line of the air-liquid interface may be moved to the middle of the step portion or to the flat portion of the mold.

In Experiment 2, in order to handle the above case, a mold with a step portion having an inner diameter of 16 mm and two kinds of filling heads having an outer circumferential diameter (outer diameter) of 15 mm and 14 mm were prepared. As in Experiment 1, it was visually confirmed whether or not the number of time of the entrainment of bubbles and movement of the contact line of the air-liquid interface of the polymer solution occurred. The evaluation was performed in the same manner as in Experiment 1 in a state after 20 trials were conducted. Table 2 shows the conditions and the evaluation results.

**Table 2**

| Test No. | Inner diameter of step portion [mm] | Outer diameter of filling head [mm] | Tapered structure | Number of times of bubbles | Movement of contact line |
|---|---|---|---|---|---|
| 7 | 16 | 17 | Provided | 1 | 15 |
| 8 | 16 | 15 | Provided | 2 | 2 |
| 9 | 16 | 14 | Provided | 2 | 0 |

According to Test No. 7, in the filling head in which the outer circumferential diameter the filling head is larger than the inner diameter of the step portion and a tapered structure is provided, the number of times of entrainment of bubbles is small. On the other hand, in a case where the filling head is separated from the mold, the movement of the contact line of the air-liquid interface was observed. In 7 portions of 15 portions in which the movement was observed, as time had passed, the contact line was moved to the flat portion of the mold and a transdermal absorption sheet could not be formed.

According to Test Nos. 8 and 9, the number of times of entrainment of bubbles was small and the number of times the movement of the contact line was observed was small. Thus, a transdermal absorption sheet could be stably formed.

### Experiment 3

In Experiment 2, in a case where the number of trials was increased more than 20 times, even in a case where the outer circumferential diameter (outside diameter) of the filling head was smaller than the inner diameter of the step portion of the mold, the movement of the contact line was observed.

In observation, in a case where the center position of the mold step and the center position of the filling head were largely misaligned, the step portion of the mold was in contact with the tip end of the filling head. The contact causes an insufficient supply of polymer solution. After the end of the supply of the polymer solution, the polymer solution was thin at the contact position.

In Experiment 3, in order to handle the above case, the supply start position of the filling head and the supply end position of the filling head were changed. At the start position, half of the total supply amount from the filling head was supplied to the mold and the supply was interrupted. The filling nozzle was separated (pulled up) from 0.3 mm mold at 5 mm/s. This position was set as the end position and half of the total supply amount from the filling head was supplied to the mold.

In Experiment 2, the gap between the step portion and the filling head was large on the opposite side of the contact position while sandwiching the center position of the step portion there between. This gap made it difficult for the polymer solution to reach the step portion and thus the entrainment of bubbles was caused.

In Experiment 3, in order to handle the above case, a tapered surface with a taper angle of 45° was provided on the outer circumferential portion of the tip end of the filling head so as to gradually reduce the diameter toward the tip. The outer diameter of the tip end of the tapered surface of the filling head was smaller than the inner diameter of the step portion, and the outer diameter of the filling head excluding the tapered surface was larger than the inner diameter of the step portion. The attempt was made to make the polymer solution to easily reach the step portion.

In the experiment, from a state in which the center position of the step of the mold matched with the center position of the filling head visually, the setting of the bench-top robot was adjusted, the positions were forced to be misaligned by 0.5 mm, and the polymer solution was supplied 10 times. Table 3 shows the conditions and the evaluation results.

**Table 3**

| Test No. | Inner diameter of step portion [mm] | Outer diameter of filling head [mm] | Tapered structure | Tapered surface | Position or filling head | Number of times of bubbles | Movement of contact line |
|---|---|---|---|---|---|---|---|
| 10 | 16 | 15 | Provided | Not provided | Constant | 4 | 4 |
| 11 | 16 | 15 | Provided | Not provided | Pulled up | 3 | 0 |
| 12 | 16 | 15 | Provided | Provided | Pulled up | 0 | 0 |
| 13 | 16 | 15 | Provided | Provided | Constant | 0 | 3 |

According to Test 10, the entrainment of bubbles and the movement of the contact line were observed. According to Test 11, even in a state in which the step portion was in contact with the filling head, by supplying the polymer solution by pulling up the filling head, the movement of the contact line of the polymer solution could be prevented.

According to Test 12, even in a state in which the filling head and the step portion were separated from each other, the tapered surface provided at the tip end of the filling head caused the polymer solution to flow until the solution reached to the step portion. In a state in which the entrainment of bubbles did not occur, and in a state in which the movement of the contact line did not occur, a transdermal absorption sheet could be formed.

According to Test 13, the entrainment of bubbles did not occur. On the other hand, the movement of the contact line was observed. According to Experiment 3, it can be assumed that the tapered structure and the tapered surface of the filling head reduce the number of times of the entrainment of bubbles and the pulling up of the filling head reduces the movement of the contact line.

### Others

The technical scope of the present invention is not limited to the scope described in the above embodiments. The configuration and the like in each embodiment can be appropriately combined in the embodiments without departing from the spirit of the present invention.

### Explanation of References

10: precursor
100: transdermal absorption sheet
102: base
102A: first surface
102B: second surface
110: protruding pattern
112: needle-like
114: needle portion
116: frustum portion
120: mold
120A: first surface
120B: second surface
122: flat portion
124: step portion
124A: corner portion
130: recessed pattern
132: needle-like recessed portion
134: tip end recessed portion
136: cup portion
150: filling head
152: head main body
154: supply pipe
154A: cylindrical pipe
154B: tapered pipe
156: jetting opening
160: inner wall surface
160A: cylindrical pipe surface
160B: tapered pipe surface
170: liquid material
200: filling head
202: head main body
204: jetting opening
206: supply pipe
206A: cylindrical pipe
206B: tapered pipe
208: inner wall surface
208A: cylindrical pipe surface
208B: tapered pipe surface
300: filling head
302: head main body
304: jetting opening
306: supply pipe
306A: cylindrical pipe
306B: tapered pipe
308: inner wall surface
308A: cylindrical pipe surface
308B: tapered pipe surface
310: tapered surface

## Claims

1. A method for producing a transdermal absorption sheet comprising:
a step of preparing a mold having a plurality of needle-like recessed portions, and a step portion which surrounds the plurality of needle-like recessed portions and is higher than a needle-like recessed portion formed surface; and
a step of supplying a liquid material for a transdermal absorption sheet from a jetting opening of a filling head to a region surrounded the step portion of the mold,
wherein the filling head includes a head main body having an inner wall surface defining a supply pipe, and the inner wall surface includes a tapered pipe surface defining a tapered pipe having a diameter gradually increased toward the jetting opening of the head main body.

2. The method for producing a transdermal absorption sheet according to claim 1,
wherein a diameter of a tip end of the head main body is smaller than an inner diameter of the step portion.

3. The method for producing a transdermal absorption sheet according to claim 2,
wherein in the step of the supplying, a supply end position of the liquid material from the filling head is further separated from the mold than a supply start position of the liquid material from the filling head.

4. The method for producing a transdermal absorption sheet according to claim 3,
wherein while the filling head is moved from the start position to the end position, the liquid material is continuously supplied.

5. The method for producing a transdermal absorption sheet according to claim 3,
wherein while the filling head is moved from the start position to the end position, the liquid material is intermittently supplied.

6. The method for producing a transdermal absorption sheet according to any one of claims 1 to 5,
wherein the head main body has a tapered surface having a diameter gradually decreased toward a tip end of the head main body.

7. The method for producing a transdermal absorption sheet according to any one of claims 1 to 6, further comprising:
a step of performing suction from a surface of the mold opposite to the needle-like recessed portion formed surface.

8. The method for producing a transdermal absorption sheet according to any one of claims 1 to 6, further comprising:
a step of forming a transdermal absorption sheet by drying the liquid material and separating the formed transdermal absorption sheet from the mold after the step of the supplying.

9. The method for producing a transdermal absorption sheet according to any one of claims 1 to 8,
wherein the liquid material is a polymer solution.

10. The method for producing a transdermal absorption sheet according to any one of claims 1 to 9, further comprising:
a step of forming a drug layer including a drug at a tip end of the needle-like recessed portion before the step of the supplying.

11. The method for producing a transdermal absorption sheet according to any one of claims 1 to 10,
wherein the mold is formed of silicone resin.

12. The method for producing a transdermal absorption sheet according to any one of claims 1 to 11,
wherein the step portion circumferentially surrounds the plurality of needle-like recessed portions, and the head main body is cylindrical.
